# EUROPEAN PATENT APPLICATION

(11) **EP 1 295 566 A1**
(43) Date of publication of application: **26.03.2003**
(21) Application number: 00940425.2
(22) Date of filing: 30.06.2000
(51) Int. Cl.: A61B 17/70

(54) **POLYAXIAL VERTEBRAL FIXING SYSTEM**

(71) Applicant: Surgival Co., S.A., 46980 Paterna, Valencia (ES)
(72) Inventor: ROBRES RUIZ, Lorenzo, E-46980 Paterna, Valencia (ES); CACERES PALO, Enric, E-46980 Paterna, Valencia (ES)
(74) Representative: Perez Bonal, Bernardo
(86) International application number: ES0000233
(87) International publication number: WO02002024

(57) **Abstract**

The insertion of the screw (1) with grooves (1.1) in the vertebra (3) takes place in a wide conical space, secured in the lip (2.1) of the bell (2) which holds the bottom end of the head (1.2) of screw (1) which as a spherical sector (1.4); the cup (4) is housed in bell (2) and incorporates the longitudinal rod (5) by an attachment screw (6) and an external screw (7) which prevents it from opening, while transverse rods (8) hold the position by means of a bottom piece (9) and a bushing (10) with a toothed crown which is complementary of a similar one in a top bushing (11) which houses the transverse rod (8).

## Description

### OBJECT OF THE INVENTION

The present invention relates to a multiaxial vertebral fixation system, from among the vertebra fixation devices using pairs of screws inserted in said vertebrae, and joined to each other by longitudinal rods and by transverse rods with means for attachment to the former rods, so that they stay in position when the system is loaded.

The invention is characterized by a special embodiment of the screws, based on a multiaxial construction as well as a receptor bell plate, a top cup incorporated to the longitudinal rod which joins screws of different vertebrae and a fixation screw.

In addition, there are fixation means which join these rods to the transverse rods, positioned by toothed crowns.

### BACKGROUND OF THE INVENTION

Devices for vertebral fusion and correction of spinal deformities have been known for a long time, involving the construction of an assembly between at least two usually consecutive vertebrae and fixation of this assembly to the selected vertebrae by at least a pair of screws.

These conventional fixation systems require great precision during the insertion of the screws in the vertebrae as the orifices for passage of the rods which join the vertebrae must be axially aligned. This can be difficult to achieve in the operating room in certain cases and in general slows down the process.

A further problem is the transverse rods which fix the position of the former rods when the system is loaded. In a conventional construction their fixation means are perpendicular to said rods, so that if the rods are not exactly parallel they can be forced as far to said perpendicular position, possibly damaging the vertebrae.

The applicant is not aware of any multiaxial insertion screws with similar coupling characteristics which allow to position the longitudinal rod independently of the orientation of the screw axis, as well as with fixation means for the transverse screws by toothed crowns that allow a non-orthogonal connection of the longitudinal rods.

### DESCRIPTION OF THE INVENTION

The invention disclosed of a multiaxial vertebral fixation system corresponds to the field of fixation means for preferably consecutive vertebrae, although the system disclosed allows its use with sufficiently distant vertebrae in such cases as when the intermediate vertebrae are greatly damaged.

Fixation is achieved conventionally by pairs of screws inserted in said vertebrae, joined to each other by the known longitudinal rods and with the assembly completed by further rods placed transverse and provided with means for attachment to the former rods, and meant to keep them in position while loaded.

The invention is characterized by a special construction in which the fully threaded mutiaxial screws with Allen heads have a large spherical sector on their heads with horizontal striations on its external surface, and with said screws being fully inserted into an internally drilled bell so that the bottom end of the head is secured by an internal lip of said internal orifice, so that the screw can be attached to the vertebra in a wide conical space.

A cup is housed on the top of said bell to cover and retain the external face of the screw head as well as to house the longitudinal rod in a transverse sense. The rod and the cup are tightened from the top of the bell by a full-thread Allen screw without head projections, which secures the rod-screw assembly, while the new coplanar screw externally secures the bell preventing its accidental opening and fixes the position of the rod.

In addition to this assembly comprising a multiaxial screw, a receptor bell, a top cup, a longitudinal rod, a cylindrical rod and finally the two top screws, one internal and one external, the invention incorporates means for fixing and securing the initial alignment of each pair of rods between vertebrae by means of further rods placed transversally and positioned by means of attachment to each other, which means are provided with toothed crowns.

The bottom irregular cylindrical piece is provided with a lateral routing with a cylindrical recess next to the axis of the piece, which is drilled perpendicular to the axis and provided on its top with a housing for a fixation Allen-head screw. The screw presses against the transverse rod and in turn the rod presses on a bushing, which is provided on its bottom face with a toothed crown and on the top with a circular recess in which is housed the transverse rod.

This bushing is placed opposite another bushing with a complementary crown at its top face and which catches the longitudinal rod as it is pushed by the top bushing, as well as being provided with a cylindrical housing in its bottom face for the longitudinal rod.

### DESCRIPTION OF THE DRAWINGS

The characteristics of the present invention will be understood more clearly in view of the accompanying drawings, where for purposes of illustration only and in a non-limiting manner the main characteristics of the invention are shown.

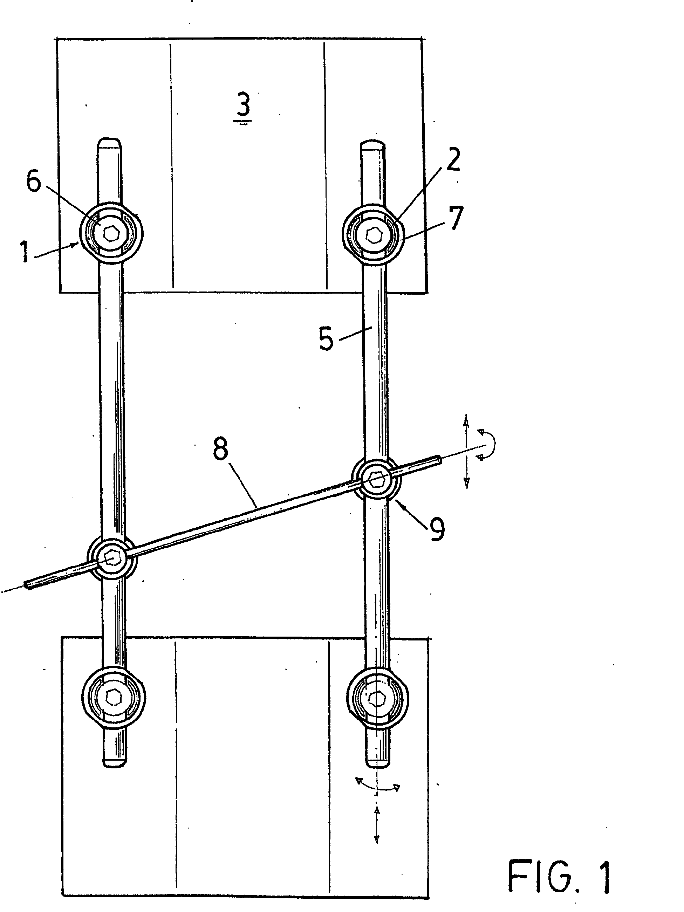

Figure 1 shows two polythene blocks which schematically represent two vertebrae with the fixation system of the invention.

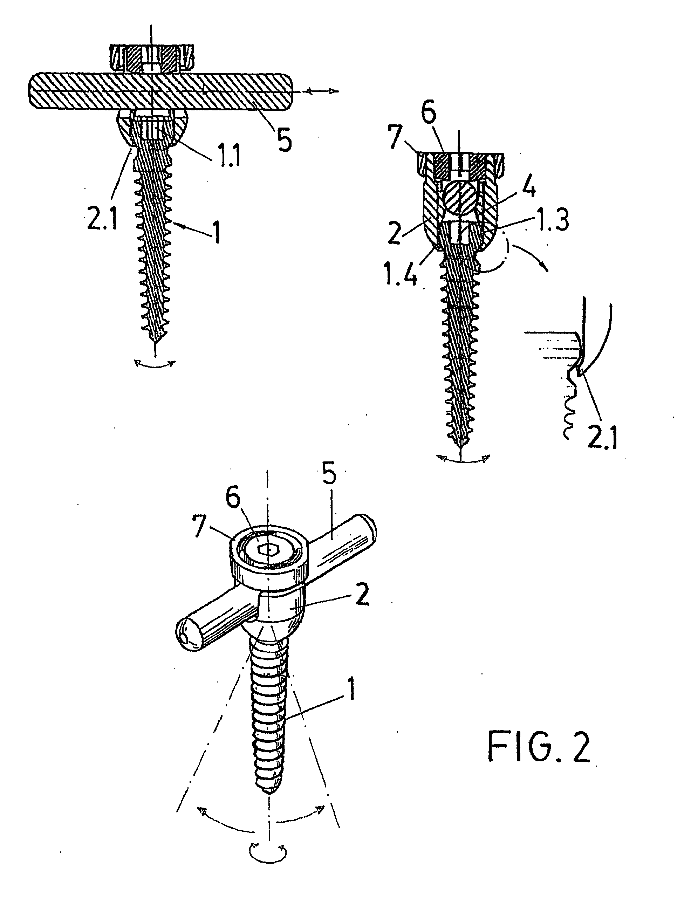

Figure 2 is a perspective view of the multiaxial screw assembly, as well as two cross sections of this assembly.

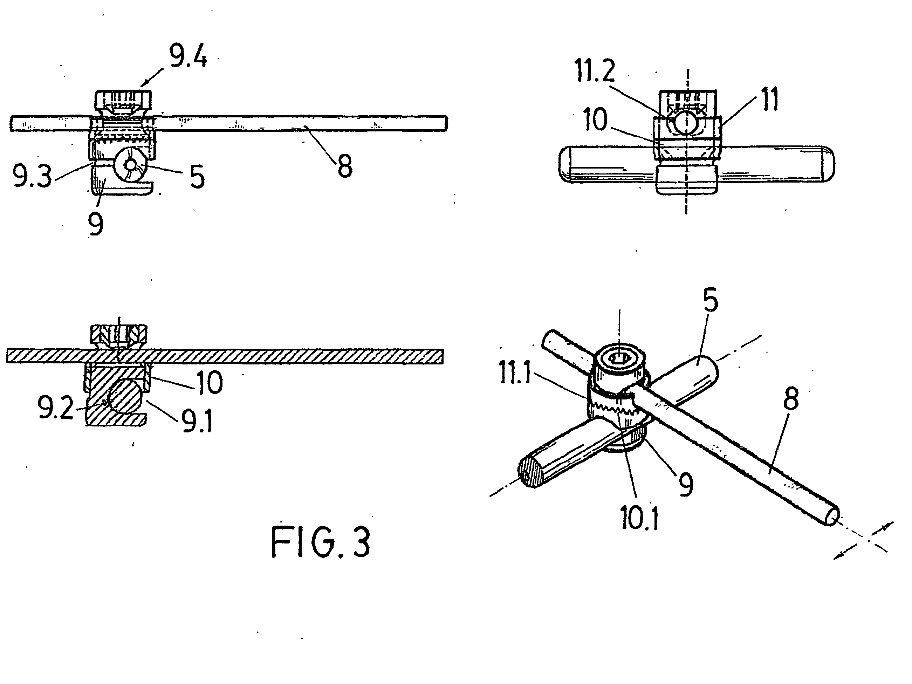

Figure 3 is a perspective view of the means for attaching the rods, as well as an elevation view and two cross sectional views of the same.

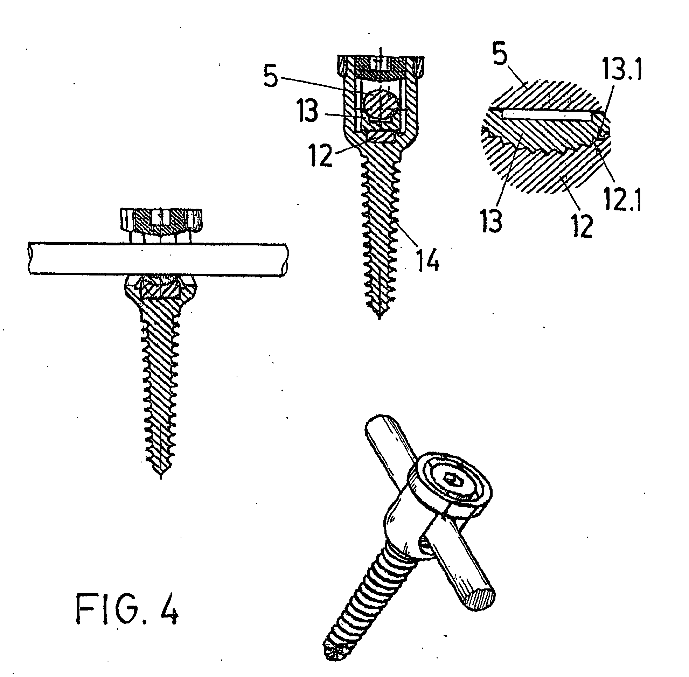

Figure 4 shows a preferred embodiment alternative in which the screw assembly is provided with an internal cradle, in a perspective view and two cross sectional views, as well as an enlarged view of the cradle.

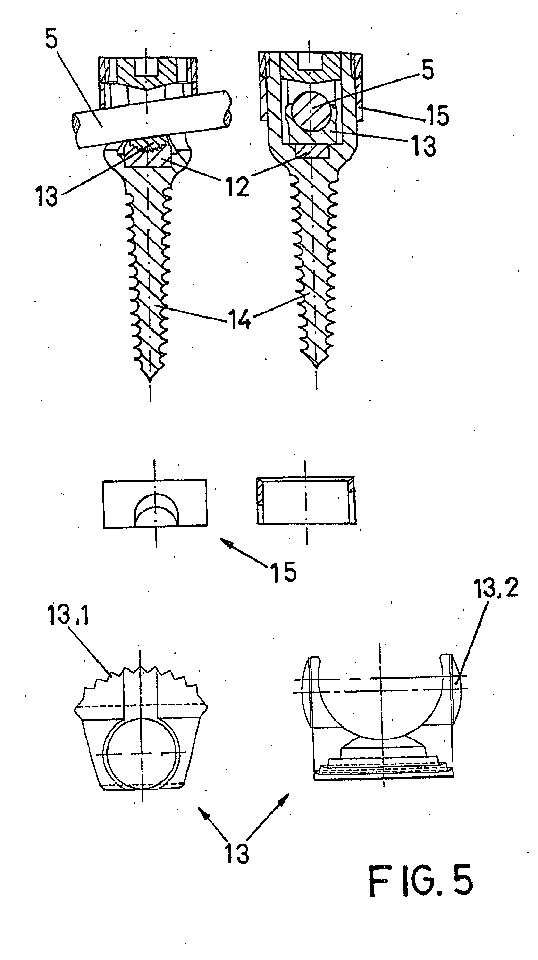

Figure 5 shows the washer which is the alternative to the former embodiment of Figure 4, with a sectional and front elevation view of the arrangement of a construction of this washer, in a section of the corresponding screw for a 10° inclination of the longitudinal rod as well as two elevation views of the spherical end cups.

### PREFERRED EMBODIMENT OF THE INVENTION

The invention disclosed for a multiaxial vertebral fixation system belongs in the field of means for fixing vertebrae by pairs of screws inserted in said vertebrae and joined to each other by longitudinal rods and with other transverse rods keeping the former rods in position, and is essentially characterized in that the screws (1) are provided with a groove (1.1) and are inserted as far as the hooked lip (2.1) at the base of the lower orifice (2.2) of a bell (2) which holds in place the bottom end of the head (1.3) of screw (1), with a spherical sector (1.4) and horizontal striations parallel to the respective cutting planes of its polar caps, so that the play of the head of screw (1) allows its insertion in the vertebra (3) in a wide conical area with a vertex external to said vertebra.

The cup (4) receives the pressure of the Allen screw (6) and is also housed in the bell (2). As it receives the gradually increasing pressure from the assembly fixation screw (7) through the longitudinal rod (5), it holds and locks in place the striation of the head (1.3) of screw (1) against the shoulder of the hooked lip (2.1), so that the two lock onto each other. The screw (7) outside the bell (2) prevents the latter from opening and presses on the longitudinal rod (5).

The means for attaching and fixing the position of each pair of longitudinal rods (5) between two vertebrae (3) consist of transverse bars (8) which are positioned by connection devices comprising a bottom plate (9). This plate is cylindrical in shape and is provided with side routings (9.1) in the form of a cylindrical recess (9.2) next to the axis of the piece, and is drilled (9.3) perpendicular to the axis and with an upper housing (9.4) for an Allen head attachment screw which presses on the transverse rod (8), while the latter in turn presses on a bushing (11). The bottom face of said bushing (11) has a toothed crown (11.1) and its top face has a circular recess (11.2) in which is housed the transverse rod (8). This bushing is placed opposite another bushing (10) which has a complementary crown (11.1) on its top face and which catches the longitudinal rod (5) as this bushing (10) is pushed on by the top bushing (11).

Alternatively, the screw (1) and the bell (2) are replaced by a single piece (14), with this alternative further incorporating a cradle (12) with a toothing (12.1) which engages the convex base (13.1) of a cylindrical cup (13), allowing to fix the rod in any position on the plane defined by the longitudinal rod and the longitudinal axis of the transpedicular screw.

This alternative can alternatively include an external washer (15) blocked by the screw (7) which fixes the assembly and provided with two diametrically opposite notches with identical or different depths which keep the rod (5) horizontal, or which allow it to be inclined between 0° and 20°.

In this alternative the cylindrical cup (13) has an open spherical top end (3.2) which acts as a cradle for the shaft (5) as well as opposing symmetrical lips (13.3) in the form of ball joints.

The essence of this invention is not affected by variations in the materials, shape, size or arrangement of the component elements, which are described in a non-limiting manner so that an expert in the field should be able to reproduce the invention.

## Claims

1. Multiaxial vertebral fixation system means among those for fixing vertebrae by pairs of screws inserted in said vertebrae and joined to each other by longitudinal rods and with other transverse rods keeping the former rods in position and with their corresponding means of attachment, essentially **characterized in that** the screws (1) are provided with a groove (1.1) and are inserted as far as the hooked lip (2.1) at the base of the lower orifice (2.2) of a bell (2) which holds in place the bottom end of the head (1.3) of screw (1), with a spherical sector (1.4) and horizontal striations, allowing to insert the screw (1) in the vertebra (3) in a wide conical space.

2. Multiaxial vertebral fixation system as claimed in claim 1, **characterized in that** the cup (4) receives the pressure of the Allen screw (6) and is also housed in the bell (2), and as it receives the gradually increasing pressure from the assembly fixation screw (7) through the longitudinal rod (5), it holds and locks in place the striation of the head (1.3) of screw (1) against the shoulder of the hooked lip (2.1), so that the two lock onto each other, while the screw (7) outside the bell (2) prevents the latter from opening and presses on the longitudinal rod

3. Multiaxial vertebral fixation system as claimed in previous claims, **characterized in that** the means for attaching and fixing the position of each pair of longitudinal rods (5) between two vertebrae (3) consist of transverse bars (8) which are positioned by connection devices comprising a bottom plate (9); this plate is cylindrical in shape and is provided with side routings (9.1) in the form of a cylindrical recess (9.2) next to the axis of the piece, and is drilled (9.3) perpendicular to the axis and with an upper housing (9.4) for an Allen head attachment screw which presses on the transverse rod (8), which in turn presses on a bushing (11). The bottom face of said bushing (11) has a toothed crown (11.1) and its top face has a circular recess (11.2) in which is housed the transverse rod (8). This bushing is placed opposite another bushing (10) which has a complementary crown (11.1) on its top face and which catches the longitudinal rod (5) as this bushing (10) is pushed on by the top bushing (11).

4. Multiaxial vertebral fixation system as claimed in previous claims, **characterized in that**, alternatively, the screw (1) and the bell (2) are replaced by a single piece (14), with this alternative also comprising a cradle (12) with a toothing (12.1) which engages the convex base (13.1) of a cylindrical cup (13), allowing to fix the rod in any position on the plane defined by the longitudinal rod and the longitudinal axis of the transpedicular screw.

5. Multiaxial vertebral fixation system as claimed in claim 4, **characterized in that** this alternative can optionally include an external washer (15) blocked by the screw (7) which fixes the assembly and provided with two diametrically opposite notches with identical or different depths which keep the rod (5) horizontal, or which allow it to be inclined between 0° and 20°, while the cylindrical cup (13) has an open spherical top end (3.2) which acts as a cradle for the shaft (5) and opposing symmetrical lips (13.3) in the form of ball joints.
